(19) Europäisches Patentamt
European Patent Office
Office européen des brevets

(11) **EP 1 559 440 B1**

(12) **EUROPEAN PATENT SPECIFICATION**

(45) Date of publication and mention
of the grant of the patent:
**23.09.2015 Bulletin 2015/39**

(51) Int Cl.:
*A61L 31/06* (2006.01)      *A61L 31/14* (2006.01)
*A61L 24/04* (2006.01)      *C08L 71/02* (2006.01)

(21) Application number: **03758990.0**

(86) International application number:
**PCT/JP2003/013773**

(22) Date of filing: **28.10.2003**

(87) International publication number:
**WO 2004/039425 (13.05.2004 Gazette 2004/20)**

(54) **VASCULAR EMBOLIZATION METERIAL**

GEFÄSSEMBOLISATIONSMATERIAL

MATERIAU D'EMBOLISATION VASCULAIRE

(84) Designated Contracting States:
**AT BE BG CH CY CZ DE DK EE ES FI FR GB GR HU IE IT LI LU MC NL PT RO SE SI SK TR**

(30) Priority: **29.10.2002  JP 2002313927**

(43) Date of publication of application:
**03.08.2005  Bulletin 2005/31**

(73) Proprietor: **TORAY INDUSTRIES, INC.**
**Tokyo 103-8666 (JP)**

(72) Inventors:
• **TABATA, Norikazu**
**Otsu-shi, Shiga 520-0822 (JP)**
• **TANAHASHI, Kazuhiro**
**Otsu-shi, Shiga 520-0801 (JP)**

• **NAKANISHI, Megumi**
**Otsu-shi, Shiga 520-2132 (JP)**

(74) Representative: **Webster, Jeremy Mark et al**
**Mewburn Ellis LLP**
**City Tower**
**40 Basinghall Street**
**London EC2V 5DE (GB)**

(56) References cited:
**EP-A1- 1 044 693      EP-A2- 0 092 918**
**WO-A1-99/22774       WO-A2-01/72280**
**WO-A2-01/80713       JP-A- 2 045 417**
**US-A- 5 635 215        US-A1- 2002 111 392**

**Description**

The present invention relates to an embolization material suitable for use in blocking a blood vessel to stop the blood flow in vivo.

It is known that if an embolization material is injected into a blood vessel prior to an incision for a surgical operation with an intension to minimize hemorrhage, hemostasis can be ensured reliably and quickly. Furthermore, aside from the purpose of preventing hemorrhage, known is the arterial embolization intended for hemostasis to stop the supply of nutrition to a tumor incapable of being excised. Moreover, known is the chemical embolization therapy in which an anticancer drug and an embolization material are administered in combination to keep the anticancer drug concentration high in a tumor.

Thanks to the development of catheters and their operation methods, an adequate embolization material can now be selectively accurately fed into the site to be blocked. Conventional intravascular embolization materials as used in these practices include liquid materials and solid materials.

Known liquid materials include organic solvents per se, and solutions obtained by dissolving monomers and polymers into organic solvents. Typical examples include the following:

Ethanol described in M. Koda, et al., Cancer, 2001, Vol. 92 (6), pages 1516-1524,
Cyanoacrylate described in the n-BCA Trial Investigators, American Journal of Neuroradiology, 2002, Vol. 23 (5), pages 748-755 Solution of ethylene-vinyl alcohol copolymer described in JP2000-502321T2 (pages 1-20).

These liquid materials have an advantage that they can virtually perfectly fill blood vessels at intended sites irrespectively of blood vessel diameters, blood vessel bifurcations and blood vessel forms.

However, they have a problem that since they are liquids with low viscosities, it is difficult to control their concentrations at injected sites. Furthermore, they have such problems that they flow to the peripheries of distal portions and that they leak into veins. Still furthermore, since an organic solvent such as dimethyl sulfoxide is used, any influence on the bodys is feared.

On the other hand, the solid materials include metallic materials, organic synthetic materials and materials derived from natural materials. Typical examples of metallic materials include the Ni-Ti coils and stents described in M. Anthony, et al., American Journal of Neuroradiology, 2000, Vol. 21 (8), pages 1523-1531.

Known typical examples of the organic synthetic materials include the following:

Poly(vinyl alcohol) particles described in C.P. Dardeyn, American Journal of Neuroradiology, 1995, Vol. 16, pages 1335-1343 Sodium acrylate-vinyl alcohol copolymer particles described in JP6-56676A (pages 1-8),
Gelatin-covered crosslinked polyacrylate particles described in US Patent 5635215 and JP6-508139T2
Ethylene-vinyl alcohol copolymer foam described in JP2000-502321T2 (pages 1-20), JP2000-505045T2 (pages 1-25) and JP2000-506514T2 (pages 1-25).

Radiation-crosslinked biodegradable synthetic hydrogels are described in US 2002/111392.

WO 01/72280 relates to injectable compositions comprising substantially hydrophilic and substantially spherical polymeric material carriers.

WO 01/80713 proposes resorbable materials which may be made from polyglycolic acid, polyvinyl alcohol, polyvinylpyrrolidone, gelatin or crosslinked hyaluronic acid.

Known materials derived from natural materials include the following:

Crosslinked starch particles described in T. Kumada, et al., Nihon-Rinsho (= Japanese Clinical Medicine), 2001, 59, Suppl., 6, pages 539-544, crosslinked gelatin particles described in JP60-20934A (pages 1-4) and JP60-222046A (pages 1-5), and alginic acid gel described in Patent No. 3103368 (pages 1-5) and JP6-329542A (pages 1-4).

These solid materials have advantages that they are easy to handle when they are indwelled or injected and that they are excellent in operation convenience, but have a problem that they cannot respond to complicated forms of blood vessels.

Especially the conventional embolization materials formed as particles have the following problems.

(1) It can happen that they cannot block blood vessels at intended sites.
(2) It can happen that they cohere together in catheters, to clog the catheters.
(3) It can happen that they cohere together in normal blood vessels on the way to the intended blood vessels and do not reach the diseased parts.
(4) It can happen that they cannot perfectly stop the blood flow though they can reduce it.

(5) In the case where a material non-degradable in vivo is used, it remains in vivo for a long period of time without being degraded or absorbed though it is not necessary to permanently stop the blood flow.

Known examples of biodegradable or bioabsorbable materials used include the following:

Poly(lactic acid) particles described in C. Witte, et al., European Journal of Pharmaceutics and Biopharmaceutics, 2001, Vol. 51, Nos. 171-181, gelatin sponge described in International Publication No. 98/03203, and crosslinked starch particles described in the aforesaid document of T. Kumada, et al.

These materials have a feature that they are hydrolyzed or degraded by enzyme in vivo. However, these materials have the above-mentioned problems (1) through (4). Furthermore, since starch particles are hydrolyzed by amylase in blood in a time period in the order of minutes, they are inadequate for long-term hemostasis and embolization. Moreover, JP5-969A discloses an embolization material composed of a copolymer obtained by copolymerizing biodegradable poly(lactic acid) or lactic acid and glycolic acid and containing a specific drug. This also has the above-mentioned problems (1) through (4).

JP5-17245B discloses that a drug is mixed with a base polymer such as poly(lactic acid) (hereinafter called PLA)-poly(ethylene glycol) (hereinafter called PEG), PLA-PEG-PLA or {poly(lactic acid/glycolic acid) copolymer} (hereinafter called PLGA)-PEG-PLGA as a block copolymer consisting of PEG, PLA or PLGA, for being slowly released, and that it is used for medicinal and pharmaceutical applications. However, the document does not teach the use as an embolization material.

The present invention provides an embolization material as defined in the accompanying claim 1.

1. The invention provides an embolization material that has a water swelling ratio of 30% or more and is degradable in a phosphate buffered saline of 37°C, being formed as virtually spherical particles with an average particle size of 50 micrometer or more. The embolization material is composed of a water insoluble polymer. When a film formed from the water insoluble polymer is saturated with water, it has an elastic modulus in tension of 1500 MPa or less.

The embolization material comprises a water insoluble poly (ethylene glycol) copolymer which is a copolymer with a structure in which a poly(ethylene glycol) derivative which is selected from a poly (ethylene glycol), methoxy poly(ethylene glycol) and multi-branched poly (ethylene glycol) and a biodegradable polymer are chemically bonded to each other, wherein the water insoluble poly(ethylene glycol) copolymer has a weight average molecular weight of 3000 to 100000, and the structure of the poly(ethylene glycol) derivative existing in the poly (ethylene glycol) copolymer has a weight average molecular weight of 2000 to 50000.

Embodiments of the invention may include:

2. An embolization material, according to item 1, wherein the film saturated with water has an elastic modulus in tension of 4 to 400 MPa.

3. An embolization material, according to item 1 or 2, wherein the elastic modulus in tension of the film saturated with water is 60% or less of the elastic modulus in tension of the film in the dry state.

4. An embolization material, according to any one of items 1 through 3, wherein the film saturated with water has a tensile elongation of 100% or more.

5. An embolization material, according to any one of items 1 through 4, which has a water swelling ratio of 100% or more.

6. An embolization material, according to any one of items 1 through 5, wherein the weight of the water insoluble polymer remaining after it is immersed in a phosphate buffered saline of 37°C for 28 hours is 80% or less of the weight of it not yet immersed.

7. An embolization material, according to item 1, wherein the water insoluble poly(ethylene glycol) copolymer is a copolymer with a structure in which a biodegradable polymer is chemically bonded to the hydroxyl groups of the poly(ethylene glycol) derivative.

8. An embolization material, according to item 1, wherein the water insoluble poly(ethylene glycol) copolymer is a mixture consisting of a poly(ethylene glycol) copolymer containing a polymer synthesized from L-lactic acid or L-lactide as the structure of the biodegradable polymer and a poly(ethylene glycol) copolymer containing a polymer synthesized from D-lactic acid or D-lactide as the structure of the biodegradable polymer.

9. An embolization material, according to any one of items 1 through 8, wherein the poly(ethylene glycol) derivative as a component of the water insoluble poly(ethylene glycol) polymer has a structure in which a compound having three or more hydroxyl groups and poly(ethylene glycol) are chemically bonded to each other.

10. An embolization material, according to any one of items 1 through 9, which is formed as particles at 37°C which have an average particle size of 50 to 2000 micrometer.

11. An embolization material, according to item 10, which has a particle size distribution width in a range of average particle size $\pm$ 100 micrometer.

12. An embolization material, according to any one of items 1 through 11, which can be swollen with at least any one of purified water, physiologic saline and water soluble X-ray contrast medium.

13. An embolization material, according to any one of items 1 through 12, which further holds a water soluble X-ray contrast medium in it.

14. An embolization material, according to any one of items 1 through 13, which has flexibility of being deformed in response to the form of the blood vessel at the time of embolization for allowing the blood flow to be stopped.

An embolizing agent may have the embolization material as set forth in any one of items 1 through 14 dispersed in a physiologic saline. An embolization method may comprise the steps of inserting a catheter percutaneously into a blood vessel of a body, to let its tip reach the site to be blocked, and injecting a solution containing the embolization material as set forth in any one of items 1 through 14 through the catheter into the site to be blocked, for blocking the blood vessel.

THE BEST MODES FOR CARRYING OUT THE INVENTION

The embolization material and the embolizing agent of this invention are therapeutic materials that are intended to easily reach the intended portion of a blood vessel and to be deformed in response to the blood vessel form near a tumor, lesion or bleeding site to positively block the blood vessel for stopping the blood flow without injuring the blood vessel, and is intended to be degraded for vanishing after the purpose has been achieved. Materials capable of satisfying these intensions have been examined intensively, and as a result, modes preferred in view of flexibility, degradability, form (particles, virtually spherical form, particle size, particle size distribution width) and compositions of raw polymers have been found.

It is preferred that the embolization material of this invention is flexible. As a flexible material, a material capable of absorbing water and swelling can be preferably used. A material being capable of absorbing water and swelling means that the material can absorb water and swell because of the absorbed water, to increase in weight and volume. If the material is formed as particles, the swelling ratio of the material owing to water can be used as a flexibility indicator. Even if the material is formed as particles or any other form, the compound or composition constituting the material can be formed into a film, and the water content or elastic modulus of the film saturated with water can be used as a flexibility indicator.

In this invention, in the case where the embolization material is formed as particles, the swelling ratio owing to water can be measured as described below.

If the diameter of a particle swollen after immersion in purified water for more than 12 hours is R, and that in the dry state is R0, the water swelling ratio (%) can be calculated from $(R^3 - R_0{}^3)/R_0{}^3 \times 100$. ($R^3$ means the third power of R, and $R_0{}^3$, the third power of $R_0$.) A micrometer can be used to observe the changes of particle sizes, and the mean value of volume changes of 10 particles can be used as the water swelling ratio. In the above, the dry state refers to a state reached by drying in vacuum for more than 12 hours subsequently to preliminary drying in air. In the examples of this invention, "an ultra depth shape measuring microscope, VK-8500" produced by Keyence was used for directly observing the particle sizes.

In view of the flexibility of being deformed in response to the inner form of a blood vessel without injuring it, the water swelling ratio is 30% or more. Especially preferred is 100% or more. In the case where the water swelling ratio is too small, even particles smaller than the inner diameter of a microcatheter cannot pass through the microcatheter.

Furthermore, in this invention, the water content can be measured using a cast film as described below.

At first, the compound or composition constituting embolization particles or an embolization material is dissolved into an organic solvent capable of dissolving it, to obtain a solution. The solution is developed on a plate with an inner diameter of 85 micrometer and dried to obtain an about 30 micrometer thick film.

The water content obtained by measuring the weight W of the film saturated with water after immersion in purified water for more than 12 hours and the weight $W_0$ of the film in the dry state for calculating the difference between the weights can also be used as an indicator of water swelling capability. It can be calculated from the following formula:

$$\texttt{Water content (\%) = (W - W_0)/W_0 x 100}$$

In view of the flexibility of being deformed in response to the form of a blood vessel without injuring the blood vessel, it is preferred that the water content of the film is 30% or more. Especially preferred is 100% or more. In the case where the water content of the film saturated with water is too low, even particles smaller than the inner diameter of a microcatheter is unlikely to pass through the microcatheter.

Moreover, in this invention, the elastic modulus values and tensile elongation values of the film saturated with water and the film in the dry state can be measured as described below. The film can be produced according to the above-mentioned method.

Test environment: Laboratory temperature 23°C, laboratory humidity 50%

Form of specimen: Strip (7.5 mm x 80 mm)

Inter-chuck distance: 20 mm

Stress rate: 10 mm/min

Except these measuring conditions, the measurement was made according to the method described in JIS K 7161 (1994). Meanwhile, in the examples of this invention, "RTM-100" produced by Orientec Corporation was used as the tensile tester.

In view of the flexibility of being deformed in response to the form of a blood vessel without injuring the blood vessel, the elastic modulus in tension of the film saturated with water is 1500 MPa or less. It is more preferred that the elastic modulus in tension is from 4 to 400 MPa. It is especially preferred that the elastic modulus in tension of the film saturated with water is 60% or less of the elastic modulus in tension of the film in the dry state. In addition, for acquiring such deformation resistance that the material is not broken even at a high pressure acting during the injection from a syringe into a blood vessel, it is especially preferred that the film saturated with water has a tensile elongation of 100% or more.

It is preferred that the embolization material of this invention is degradable in vivo. An indicator of the degradability in vivo can be the weight loss of particles in a phosphate buffered saline of 37°C. It is preferred that the weight of the embolization material of this invention remaining after it is immersed in a phosphate buffered saline of 37°C for 28 days is 80% or less of the weight of it not yet immersed. It is more preferred that the weight of it remaining after immersion for 28 days is 50% or less of the weight of it not yet immersed. The phosphate buffered solution can be obtained, for example, by diluting the phosphate buffered saline (pH 7.4, 10-fold concentrated) produced by Nacalai Tesque, Inc. to 10 times. In the examples of this invention, about 200 mg (dry weight W0) of an embolization material was dispersed into 10 mL of the phosphate buffered saline in a test tube, and the test tube was rotated at a rate of one revolution per 2 seconds by a rotator, for shaking the content of the test tube in an environment of 37°C. After lapse of a predetermined period of time, the water soluble component was removed using a micropipette, and subsequently the washing of the residue with purified water and the removal of the water soluble component were repeated three times. The residue was dried in air and dried in vacuum, and its weight W was measured.

The embolization material of this invention is formed as particles at 37°C, specifically as virtually spherical particles. It is more preferred that the average particle size and the particle side distribution width are in respective specific ranges as described later.

An embolization material formed as particles can be produced, for example, by the following method.

The polymer (the detail is described later) used as a raw material of the embolization material is dissolved into, for example, dichloromethane, chloroform, ethyl acetate, isopropyl ether or the like, to obtain a solution. It is dispersed into a water phase containing a surfactant, protective colloid agent or the like, and a publicly known method of drying in an O/W emulsion or W/O emulsion, a similar method, spray-drying method, or the like is used to obtain an embolization material formed as particles.

As another method, the raw material of the embolization material is dissolved into a water miscible organic solvent such as methanol, acetone or tetrahydrofuran, to obtain a solution, and the organic solvent used as a component of the solution is substituted by water or water containing a surfactant, to obtain an embolization material formed as particles. The surfactant or protective colloid agent used in this case is not especially limited, if it can form a stable oil/water emulsion. Examples of the materials include anionic surfactants (sodium oleate, sodium stearate, sodium lauryl sulfate, desoxycholic acid, etc.), nonionic surfactants (polyoxyethylene sorbitan fatty acid ester, polyoxyethylene castor oil derivative, etc.), poly (vinyl alcohol), polyvinylpyrrolidone, carboxymethyl cellulose, lecithin, gelatin, etc. Plural compounds can also be selected from among them for use in combination.

Furthermore, as the surfactant or protective colloid agent, a water soluble A-B type block copolymer in which A denotes a biodegradable polymer while B denotes methoxy polypoly(ethylene glycol) can be preferably used. Among the surfactants and protective colloid agents, poly(vinyl alcohol), carboxymethyl cellulose and gelatin can be especially preferably used.

A preferred concentration of the surfactant or protective colloid agent can be selected in a range from 0.01 to 20 wt% as an aqueous solution. A more preferred range is from 0.05 to 10 wt%.

The particles produced like this are generally virtually spherical particles, but some amorphous particles may be contained. For the purpose of removing such particles or for the purpose of classification, plural sieves with adequate mesh sizes can be used to obtain particles with an intended average particle size and an intended particle size distribution. In this case, the particles to be sieved can be either dry particles or wet particles immersed in water.

In the case where the embolization material of this invention is formed as particles, the particle size and particle size distribution can be measured by an electric resistance method or light scattering method.

As a means for using the electric resistance method, Coulter Multisizer II or III produced by Scientific Instruments can be used. As a means for using the light scattering method, "MICROTRAC HRA-X100" produced by Leeds and Northrup can be used.

In the former case, since measurement is made in a physiologic saline, measurement can be made in an environment close to the inside of a blood vessel. In the latter case, measurement is made in purified water. In either case, the value of volume average is employed as the average particle size.

The average particle size of the embolization material of this invention is 50 micrometer or more. More preferred is 60 micrometer or more. On the other hand, 2000 micrometer or less is preferred. More preferred is 1500 micrometer or less. In the case where the average particle size is too small, a site other than the intended blood vessel may be blocked. Considering the diameter of the actual blood vessel to be blocked, it is preferred that the average particle size is in a range between said lower limit and said upper limit. It is further preferred that the average particle size is selected as required in response to the diameter of the blood vessel to be blocked.

For the purpose of realizing more reliable blocking, it is preferred that the particle size of the embolization material of this invention is uniform. For example, it is preferred that the particle size distribution width is in a range of average particle size $\pm$ 100 micrometer. More preferred is a range of average particle size $\pm$ 50 mictometer. Meanwhile, the particle size distribution width refers to a particle size range from D1 to D99 for average particle size D50 in terms of volume.

The embolization material of this invention is formed as virtually spherical particles, as virtually spherical particles allow perfect blocking in a blood vessel.

The embolization material of this invention contains a synthetic polymer artificially synthesized and it is preferred that the embolization material is mainly composed of a synthetic polymer.

Examples of the synthetic polymer in this invention include a water insoluble poly(ethylene glycol) copolymer and non-crosslinked poly (propylene fumarate) synthesized from fumaric acid and propylene glycol. At first, the water insoluble poly (ethylene glycol) copolymer is described.

A water insoluble poly(ethylene glycol) copolymer is poly(ethylene glycol), a poly(ethylene glycol) derivative such as methoxy poly(ethylene glycol) or multi-branched poly(ethylene glycol), a block copolymer obtained using a poly(ethylene glycol) derivative as a raw material component, or a stereo complex forming block copolymer in which two or more block copolymers physically interact with each other, etc., and is insoluble in water. A copolymer in which physical interaction with poly (ethylene glycol) or any of its derivatives causes insolubilization in water, such as polyrotaxane formed from cyclodextrin and poly(ethylene glycol) can also be used. Multi-branched poly(ethylene glycol) is a compound having a structure in which a compound with three or more hydroxyl groups such as glycerol, polyglycerol, pentaerythritol or polypentaerythritol and poly(ethylene glycol) are chemically bonded to each other. Particularly, tri-branched "Sunbright GL," tetra-branched "Sunbright PTE" and octa-branched "Sunbright HGEO (respectively produced by NOF Corp.) can be preferably used. Originally poly (ethylene glycol) is soluble in water. So, in this specification, the water insoluble poly (ethylene glycol) copolymer means that a structure other than the structure of poly(ethylene glycol) contributes to ensure that the entire poly (ethylene glycol) polymer containing the structure of poly(ethylene glycol) is not dissolved into water. Particularly it means that when said material is immersed in water at 23°C, it is not dissolved into water within 30 minutes.

The molecular weight of the poly(ethylene glycol) structure portion contained in the poly(ethylene glycol) copolymer is a weight average molecular weight in a range from 2000 to 50000. If the molecular weight is in this range, the embolization material becomes homogeneously biodegradable and it does not happen that the poly(ethylene glycol) produced from the copolymer degraded in vivo is unlikely to be discharged outside the body. Furthermore, the weight average molecular weight of the poly(ethylene glycol) copolymer is in a range from 3000 to 100000.

It is preferred that the water insoluble poly(ethylene glycol) copolymer is a copolymer having a structure in which a poly(ethylene glycol) derivative (hereinafter called the block B) and a biodegradable polymer (hereinafter called the block A) are chemically bonded to each other. Furthermore, a copolymer having a structure in which the block A is chemically bonded to the hydroxyl groups of the block B is more preferred. As for the block bonding types, the following structures can be preferably used.

A copolymer in which a biodegradable polymer is chemically bonded to the hydroxyl group at one end of poly (ethylene glycol) (A-B type block copolymer)

A copolymer in which a biodegradable polymer is chemically bonded to the hydroxyl groups at both the ends of poly(ethylene glycol) (A-B-A type block copolymer)

A copolymer in which poly(ethylene glycol) and a biodegradable polymer are alternately bonded to each other {(A-B)n type multi-block copolymer}

A multi-branched copolymer having a structure in which a biodegradable polymer is chemically bonded to the three or more hydroxyl groups of multi-branched poly(ethylene glycol) wherein a compound with three or more hydroxyl groups such as glycerol, polyglycerol, pentaerythritol or polypentaerythritol as a poly(ethylene glycol) derivative and poly(ethylene glycol) are chemically bonded to each other (An-B type block copolymer)

Furthermore, the weight average molecular weight of the poly(ethylene glycol) derivative is from 2000 to 50000 while the weight average molecular weight of the aforesaid block copolymer is from 3000 to 100000.

With regard to the A-B-A type block copolymer, A-B type block copolymer, An-B type block copolymer and (A-B)n type multi-block copolymer, if the average molecular weight is too small, the copolymer is likely to be gelled, and when it is injected into a blood vessel, it can happen that the copolymer adheres to the catheter or the blood vessel, not allowing the blood vessel to be blocked at the intended site. On the other hand, if the average molecular weight is too large, there may be such a case where it takes a long time for the material with a large average molecular weight to be degraded in vivo.

The average molecular weight of a water insoluble poly(ethylene glycol) copolymer can be measured by means of gel permeation chromatography (GPC).

In the examples of this invention, the following method was used.

Column: TSK gel XL series (inner diameter 7. 8 mm, length 30 cm, produced by Tosoh Corp.)

Eluting solution: Chloroform

Column temperature: 35°C

Flow velocity: 1.0 ml/min

Detection method: Refractive index (RI8010 Refractometer: Produced by Tosoh Corp.)

Calibration curve: Prepared by using respective polystyrene standard samples with average molecular weights of 1,110,000, 707,000, 397,000, 189,000, 98,900, 37,200, 17,100, 9490, 5,870, 2,500 and 1,050 and 500

Data processing: Class Vp Data Analysis Workstation (produced by Shimadzu Corp.)

In view of uniform degradability, it is preferred that the water insoluble poly(ethylene glycol) copolymer has such a narrow molecular weight distribution that the ratio of weight average molecular weight (Mw) to number average molecular weight (Mn), i.e., Mw/Mn of the copolymer calculated from the peak measured by means of GPC is 2 or less.

With regard to the water insoluble poly(ethylene glycol) copolymer of this invention, a method for producing an A-B-A type block copolymer is exemplified below. Poly(ethylene glycol) is synthesized by polymerizing ethylene oxide by a conventional method or obtained as a commercially available product. The average molecular weight of poly(ethylene glycol) is, as described above, in a range from 2000 to 50000. As the poly (ethylene glycol) with a specified average molecular weight, products commercially available under trade names of "Macrogol" (produced by Sanyo Chemical Industries, Ltd.) and "Sunbright" (produced by NOF Corp.) can be preferably used. In the case where a ring-opening polymerization method is used to synthesize from ethylene oxide, it is preferred that the molecular weight distribution of the synthesized poly(ethylene glycol) is narrow.

Next, the copolymerization between poly(ethylene glycol) (the block B) and a raw material (for example, a monomer such as lactic acid or glycolic acid, or cyclic dimer such as lactide or glycolide) of a biodegradable polymer (the block A) described later is performed using an adequate catalyst described later. For example, in the case where a hydroxycarboxylic acid such as lactic acid or glycolic acid is polymerized, a condensation polymerization method can be preferably used, and in the case where a cyclic compound such as lactide or glycolide is polymerized, a ring-opening polymerization method can be preferably used. The produced A-B-A type copolymer is purified by a fractional precipitation method. That is, in the case where the respective structures of the block A and the block B exist respectively alone as polymers, the obtained A-B-A type copolymer is dissolved into an organic solvent capable of dissolving the respective polymers (hereinafter such a solvent is called a good solvent). While the solution is stirred, an organic solvent capable of dissolving either of the polymers but incapable of dissolving the other polymer (hereinafter such a solvent is called a poor solvent) is added dropwise into the polymer having the structure of block A and the polymer having the structure of block B, and the produced precipitate is taken out of the system. If this operation is repeated, a copolymer with a narrow molecular weight distribution, i.e., an A-B-A type copolymer with a small Mw/Mn ratio value can be produced.

After dropwise addition of a poor solvent, a precipitate is produced as a white turbid material. If the temperature of the white turbid material is changed to once dissolve the precipitate and is again slowly returned to the original temperature, to produce a precipitate, the fractionation accuracy can also be raised.

The good solvent used in said fractional precipitation method can be decided adequately depending on the polymers, and examples of it include tetrahydrofuran, halogen-based organic solvents (dichloromethane, chloroform), acetone, methanol and mixed solvents consisting of the foregoing.

The amount of the good solvent used depends on the added amounts of raw materials and the chemical composition of the copolymer. Usually the amount is from 1 to 50 wt% as the concentration in a copolymer solution. A preferred range is from 1 to 25 wt%.

The poor solvent used in said fractional precipitation method can be adequately decided depending on the polymers, but an alcohol-based organic solvent or a hydrocarbon-based organic solvent is preferred.

In said condensation polymerization method or ring-opening polymerization method, a polymerization reactor with stirring blades is charged with a poly(ethylene glycol) derivative with a predetermined average molecular weight as a raw material and the raw material of a biodegradable polymer in dry air or in a dry nitrogen stream, and heated, and the mixture is stirred together with a catalyst, to obtain the intended product. As another method, a vented double-screw kneading extruder or an apparatus having similar stirring and feeding functions is used to stir, mix and degas the raw material of the biodegradable polymer together with the catalyst, and the produced A-B-A type copolymer is continuously taken out for accomplishment of polymerization.

The raw material of the biodegradable polymer is one or more selected from the following examples of compounds.

a-hydroxy acids (for example, lactic acid, glycolic acid, 2-hydroxybutyric acid, 2-hydroxyvaleric acid, 2-hydroxycapric acid, 2-hydroxycaprylic acid, etc.), cyclic dimmers of a-hydroxy acids (for example, lactide, glycolide, etc.), hydroxydicarboxylic acids (for example, malic acid, etc.), cyclic esters such as trimethylene carbonate, epsilon-caprolactone, 1,4-dioxanone, 1,4-dioxepane-7-one

Among the cyclic dimmers of a-hydroxy acids, lactide and glycolide are preferred. Among the hydroxydicarboxylic acids, malic acid is preferred. In the case where two or more raw materials of biodegradable polymers are used, it is preferred to combine lactic acid (or lactide) and glycolic acid (or glycolide), and it is preferred that the ratio by weight of lactic acid to glycolic acid is from 100:0 to 50:50. Furthermore, in the case where two or more raw materials of biodegradable polymers are used, a combination consisting of lactic acid (or lactide) and epsilon-caprolactone is preferred, and it is preferred that the ratio by weight of lactic acid to epsilon-caprolactone is from 100:0 to 40:60. In the above, in the case of a compound optically active in the molecule such as lactic acid or lactide, any of D-form, L-form or a mixture consisting of D-form and L-form can be used.

With regard to preferred combinations constituting A-B type, A-B-A type, AnB type (n > 3) or (A-B)n type copolymer, as the biodegradable block A, preferably used are poly(lactic acid), poly(lactic acid/glycolic acid), poly(lactide/glycolide), poly(lactic acid/epsilon-caprolactone), poly(lactide/epsilon-caprolactone), poly(glycolic acid/epsilon-caprolactone), poly(glycolide/epsilon-caprolactone), poly(lactic acid/trimethylene carbonate), poly(lactide/trimethylene carbonate), poly(glycolic acid/trimethylene carbonate) and poly (glycolide/trimethylene carbonate). Furthermore, for example, if a poly(ethylene glycol) polymer containing L-lactide structure and a poly (ethylene glycol) polymer containing D-lactide structure such as a combination consisting of poly(L-lactide)-PEG and poly(D-lactide)-PEG are blended to form a stereo complex, it can be preferably used, since it is excellent in thermal stability. In the above, "/" indicates comonomers.

The catalyst used for polymerization is not especially limited if it is a catalyst used for ordinary polymerization for producing polyesters. Examples of the catalyst include tin halides such as tin chloride, tin salts of organic acids such as tin 2-ethylhexanoate, organic tins such as dibutyltin oxide, dibutyltin dichloride, dibutyltin dilaurate and dibutyltin maleate (polymer), etc. Other examples include organic alkali metal compounds such as diethylzinc, zinc lactate, iron lactate, dimethylaluminum, calcium hydride and butyllithium, metal porphyrin complex, metal alkoxides such as diethylaluminum methoxide, etc.

The water insoluble poly (ethylene glycol) copolymer has been explained above, and the embolization material containing it can further contain, as required, a polymer other than.said ingredients to such an extent that the effects of the invention are not impaired.

In this invention, in the case where the embolization material formed as particles is used in actual therapy, it is preferred to use it as an embolizing agent with the particles dissolved in an aqueous liquid, preferably a physiologic saline. A femoral artery is punctured with a double needle through the skin of the body, and subsequently the inner needle is removed while the outer cylinder is allowed to indwell in the cavity of the blood vessel. Then, a guide wire is inserted into the blood vessel through it. Using it as an axis, a guiding catheter and a microcatheter are inserted into the blood vessel. Observing an X-ray image, the tip of the microcatheter is made to reach an intended blood vessel of, for example, a hepatic artery, gastroduodenal artery, mesenteric artery, gastric artery, uterine artery, internal carotid artery, communicating artery, basilar artery, cerebral artery, cerebellar artery, etc., and subsequently, a syringe containing an embolizing agent with particles dispersed is attached to the catheter for injection. A method of mixing an X-ray contrast medium with the embolizing agent to facilitate observation under fluoroscopy can be preferably used.

The embolizing agent of this invention can be used as it is or as dispersed in an adequate dispersion medium or in a contrast medium such as an iodine addition product obtained from poppy seed oil. As the contrast medium, a publicly known one can be used, and either an ionic contrast medium or a non-ionic contrast medium can be used. Particular examples of it include "Iopamiron" (produced by Schering AG), "Hexabrix" (produced by Eiken Chemical Co., Ltd.), "Omnipaque" (produced by Daiichi Pharmaceutical Co., Ltd.), "Urografin" (produced by Schering AG), "Iomeron (produced by Eisai Co., Ltd.), etc. The embolizing agent of this invention and a contrast medium can be mixed before use, and the mixture can be injected into a predetermined site. If the water swelling capability is high, the contrast medium is partly held inside the embolization material together with water, to express the contrast effect. Examples of the dispersion medium include solutions with a dispersing agent (for example, polyoxysorbitan fatty acid ester, carboxymethyl cellulose, etc.), preservative (for example, methylparaben, propylparaben, etc.), or isotonic agent (for example, sodium chloride, mannitol, glucose, etc.) dispersed in distilled water for injection, and vegetable oils such as sesame oil and corn oil. The dispersed embolization material is administered from an adequate artery into a tumor-feeding artery using the inserted catheter while an angiographic agent is used for monitoring. Furthermore, an antiseptic, stabilizer, isotonic agent, solubilizing agent, dispersing agent, excipient, etc. usually added to an injection can also be added to the embolizing agent.

The embolizing agent of this invention can also be used together with an oily contrast medium such as an iodine addition product obtained from poppy seed oil (Lipiodol Ultra-Fluid). Moreover, it can also be used together with an iodine addition product obtained from poppy seed oil and an anticancer drug such as Smancs, neocarzinostatin, mitomycin-C, adriamycin, irinotecan hydrochloride, fluorouracil, epirubicin hydrochloride, cisplatin or vinblastine sulfate.

The embolization material of this invention can achieve the object of this invention, even if it does not contain a medicinally effective ingredient. However, for the purpose of giving a further effect, it can also contain a medicinally effective ingredient. The medicinally effective ingredient is not especially limited, if it has a known medicinal effect. Examples of the medicinally effective ingredient include anticancer drugs (for example, Smancs, neocarzinostatin, mitomycin-C, adriamycin, irinotecan hydrochloride, fluorouracil, epirubicin hydrochloride, cisplatin, paclitaxel, calcium leucovorin, vinblastine sulfate, altretamine, bleomycin, doxorubicin hydrochloride, Picibanil, Krestin, lentinan, cyclophosphamide, thiotepa, tegafur, vinblastine sulfate, pirarubicin hydrochloride), vascularization inhibitors, steroid hormones, hepatic disease drugs, arthrifuges, antidiabetic agents, drugs for circulatory organs, hyperlipidemia drugs, bronchodilators, antiallergic drugs, drugs for digestive organs, antipsychotic drugs, chemical therapeutic agents, antioxidants, peptide-based drugs, protein-based drugs (for example, interferon), etc.

This invention is described below in further detail based on examples.

Examples

[Example 1]

Under a nitrogen stream, 40.3 g of L-lactide (produced by Purac Biochem) and 17.3 g of dehydrated poly (ethylene glycol) with an average molecular weight of 20000 (produced by Sanyo Chemical Industries, Ltd.) were molten and mixed at 140°C in a flask, and 8.1 mg of tin dioctanoate (produced by Wako Pure Chemical Industries, Ltd.) was added. Then, a reaction was performed at 180°C, to obtain an A-B-A type copolymer (PLA-PEG-PLA). The obtained copolymer was dissolved into chloroform, and the solution was added dropwise into a very excessive amount of methanol, to obtain a white precipitate. The weight average molecular weight by the GPC method was about 70000.

The aforesaid purified copolymer was dissolved into dichloromethane, and by the method of drying in an O/W emulsion, spherical particles were obtained. The spherical particles were dried in vacuum, and fractionated using a nylon mesh. The fractionated particles were immersed in a physiologic saline, to obtain a dispersion containing spherical particles. The particle size distribution was measured using "MICROTRAC HRA-X100" produced by Leeds and Northrup, to obtain a volume average particle size of about 330 micrometer and a distribution width of average particle size $\pm$ 40 micrometer. The particle size was directly observed using an ultra depth shape measuring microscope, "VK-8500" produced by Keyence, and the water swelling ratios of ten particles with a diameter of about 330 micormeter immersed in purified water were measured, and a water swelling ratio of 130% was obtained.

Furthermore, a dichloromethane solution containing 5 wt% of the aforesaid purified copolymer was developed on a plate, to obtain an about 30 micrometer thick cast film. The film was immersed in purified water for 24 hours, and it was found to have a water content of 81%, an elastic modulus in tension of 184 MPa and a tensile elongation of 240%. Meanwhile, the elastic modulus in tension of the cast film in the dry state was 230 MPa.

At first, catheter passing capability was examined. The spherical particle dispersion was injected from a syringe into a catheter, Mass Transit (overall length about 1400 mm, length of tip portion 180 mm, inner diameter of tip portion about 680 micrometer) produced by Cordis. It could be injected without any resistance. Furthermore, a small amount of a physiologic saline was injected, and subsequently the catheter was dissected in the longitudinal direction to visually observe the inside of the catheter. No remaining spherical particle was observed at all.

Next, the blocking of a thin catheter used as a blood vessel model was examined. The spherical particle dispersion was injected from a syringe into a catheter, Spinnaker 1.5F (overall length about 1650 mm, length of tip portion 150 mm, inner diameter of tip portion about 280 micrometer) produced by Boston Scientific. Immediately after start of injection, a strong resistance acted not to allow injection. No particle that passed through the tip portion could be seen.

From the above, it can be seen that particles with a uniform particle size and made flexible due to water absorption and swelling passed through a microcatheter (catheter with an inner diameter of about 680 micrometer) without causing cohesion or clogging, and on the other hand, that they could reliably block a fine catheter (catheter with an inner diameter of about 280 micrometer) used as a blood vessel model. Furthermore, since the particles were swollen due to absorbed water, they were unlikely to injure the blood vessel and could be deformed in response to the form of the blood vessel, to allow perfect blocking.

Said spherical particles were added into a phosphate buffered saline (pH 7.3) (produced by Nacalai Tesque, Inc.), and after lapse of 28 days at 37°C, the remaining weight in comparison with the weight before treatment was obtained and found to be 75%.

[Comparative Example 1]

Under a nitrogen stream, 40.3 g of L-lactide (produced by Purac Biochem) and 8.1 mg of tin dioctanoate (produced by Wako Pure Chemical Industries, Ltd.) were added into a flask and a reaction was performed at 140°C to obtain poly(L-lactide). The obtained polymer was dissolved into chloroform, and the solution was added dropwise into a very excessive amount of methanol, to obtain a white precipitate. The weight average molecular weight obtained by the GPC method was about 70000.

The obtained polymer was used to obtain a spherical particle dispersion of poly(L-lactide) by the same method as described for Example 1. The volume average particle size in a physiologic saline was about 300 micrometer, and the distribution width was average particle size ± 40 micrometer. Furthermore, in the observation using a microscope, the particle size of the particles saturated with water was not different from that of the particles in the dry state, and a water swelling ratio was found to be 0%.

Furthermore, a dichloromethane solution containing 5 wt% of the aforesaid purified copolymer was developed on a plate, to obtain an about 30 micrometer thick cast film. The film was immersed in purified water for 24 hours, and it was found to have a water content of 2%, an elastic modulus in tension of 1570 MPa and a tensile elongation of 4%. The elastic modulus in tension of the cast film in the dry state was 1550 MPa.

The spherical particle dispersion of poly(L-lactide) was injected from a syringe into a catheter, Mass Transit (overall length about 1400 mm, length of tip portion 180 mm, inner diameter of tip portion about 680 micrometer) produced by Cordis. Immediately after start of injection, a strong resistance acted not to allow injection. Some particles passed through the tip portion, but most of the particles could not pass through the microcatheter.

Meanwhile, said spherical particles were added to the phosphate buffered saline (pH 7.3), and after lapse of 28 days at 37°C, the remaining weight in comparison with the weight before treatment was obtained and found to be 98%.

The particles of the embolization material of this comparative example could not be swollen with water and did not contain a poly(ethylene glycol) copolymer. So, probably because they were likely to adhere and cohere together in the catheter and blood vessel, even the particles smaller than the inner diameter of the microcatheter could not pass through the microcatheter.

[Example 2]

Under a nitrogen stream, 23.1 g of L-lactide, 9.1 g of epsilon-caprolactone and 23.1 g of poly(ethylene glycol) (dehydrated) with an average molecular weight of 8000 were mixed in a flask, and the mixture was molten and mixed at 140°C. Then, 8.1 mg of tin dioctanoate was added to perform a reaction at 180°C, for obtaining an A-B type copolymer {P(LA/CL)-PEG)}. The obtained copolymer was dissolved into chloroform, and the solution was added dropwise into a very excessive amount of methanol, to obtain a white precipitate. The weight average molecular weight by the GPC method was about 22000.

The aforesaid purified copolymer was dissolved into dichloromethane, and the method of drying in an O/W emulsion was used to obtain spherical particles. The spherical particles were dried in vacuum and fractionated using a nylon mesh. The fractionated particles were immersed in a physiologic saline to obtain a spherical particle dispersion. From the particle side distribution measurement, it was found that the volume average particle size was about 100 micrometer and that the distribution width was average particle size ± 40 mm. The water swelling ratios of ten particles with a diameter of about 100 micrometer immersed in purified water were measured, and a water swelling ratio of 37% was obtained.

Furthermore, according to the same method as described for Example 1, a film was obtained and found to have a water content of 40%, an elastic modulus in tension of 47 MPa and a tensile elongation of 15%. The elastic modulus in tension of the cast film in the dry state was 200 MPa.

A 24G indwelling needle was inserted into a femoral vein of each 10-week old rat anesthetized with Nembutal, and subsequently a spherical particle dispersion with 60 mg of the particles dispersed in 1 mL of a physiologic saline was injected. Two weeks later, the lungs were observed in appearance and tissue sections were prepared. The spherical particle dispersion was injected into four rats respectively and their tissue sections were observed. In all the four rats, pulmonary infarction was observed.

[Example 3]

Under a nitrogen stream, 21.6 g of L-lactide (produced by Purac Biochem), 5.8 g of glycolide (produced by Purac Biochem) and 28.8 g of dehydrated poly (ethylene glycol) with an average molecular weight of 20000 (produced by Sanyo Chemical Industries, Ltd.) were mixed in a flask, and the mixture was dissolved and mixed at 140°C. Then, at 180°C, 8.1 mg of tin dioctanoate (produced by Wako Pure Chemical Industries, Ltd.) was added to perform a reaction for obtaining poly(L-lactide/glycolide)-poly(ethylene glycol)-poly(L-lactide/glycolide) copolymer. The copolymer was dissolved into chloroform and the solution was added dropwise into a very excessive amount of methanol, to obtain a white precipitate. The weight average molecular weight by the GPC method was 42000.

A dichloromethane solution containing 5 wt% of the obtained purified copolymer was developed on a plate to obtain an about 30 micrometer thick cast film. The film was immersed in purified water for 24 hours, and it was found to have a water content of 215%, an elastic modulus in tension of 7 MPa and a tensile elongation of 156%. The elastic modulus in tension of the cast film in the dry state was 200 MPa.

The aforesaid purified copolymer was dissolved into dichloromethane, and the method of drying in an O/W emulsion was used to obtain spherical particles. The spherical particles were dried in vacuum and then fractionated using a nylon mesh. The fractionated particles were immersed in a physiologic saline, to obtain a spherical particle dispersion with a volume average particle size of 350 micrometer and a distribution width of average particle size ± 40 micrometer. The water swelling ratios of ten particles with a diameter of about 350 micrometer immersed in purified water were measured, and a water swelling ratio of 188% was obtained.

According to the same method as described for Example 1, a film was obtained and found to have a water content of 215%, an elastic modulus in tension of 7 MPa and a tensile elongation of 156%. The elastic modulus in tension of the cast film in the dry state was 200 MPa.

The aforesaid spherical particle dispersion was injected from a syringe into a catheter, Mass Transit (overall length about 1400 mm, length of tip portion 180 mm, inner diameter of tip portion about 680 micrometer) produced by Cordis. It could be injected without any resistance. Subsequently, the catheter was dissected in the longitudinal direction and the inside of the catheter was visually observed. No remaining spherical particle was observed at all.

Next, the blocking of a thin catheter used as a blood vessel model was examined. The spherical particle dispersion was injected from a syringe into a catheter, Spinnaker 1. 5F (overall length about 1650 mm, length of tip portion 150 mm, inner diameter of tip portion about 280 micrometer) produced by Boston Scientific. Immediately after start of injection, a strong resistance acted not to allow injection. No particle that passed through the tip portion was observed.

Meanwhile, said spherical particles were added into the phosphate buffered saline (pH 7.3), and after lapse of 28 days at 37°C, the remaining weight in comparison with the weight before treatment was obtained and found to be 40%.

Moreover, said nylon mesh was used for fractionation, and a spherical particle dispersion with a volume average particle size of about 90 micrometer and a particle size distribution width of ±50 micrometer in a physiologic saline was obtained. Then, a 24G indwelling needle was inserted into a femoral vein of each 10-week old rat anesthetized with Nembutal, and a spherical particle dispersion with 60 mg of the particles dispersed in 1 mL of a physiologic saline was injected. Two weeks later, the lungs were observed in appearance, and tissue sections were prepared. The spherical particle dispersion was injected into four rats respectively, and the tissue sections were observed. In all the four rats, pulmonary infarction was observed.

[Example 4]

Under a nitrogen stream, 19.2 g of L-lactide (produced by Purac Biochem), 9.6 g of glycolide (produced by Purac Biochem) and 28.8 g of dehydrated methoxy poly (ethylene glycol) with an average molecular weight of 20000 (produced by Sanyo Chemical Industries, Ltd.) were mixed in a flask, and the mixture was dissolved and mixed at 140°C. Then, 8.1 mg of tin dioctanoate (produced by Wako Pure Chemical Industries, Ltd.) was added at 180°C to perform a reaction, for obtaining poly(L-lactide/glycolide)-poly(ethylene glycol) copolymer. The copolymer was dissolved into chloroform, and the solution was added dropwise into a very excessive amount of methanol, to obtain a white precipitate. The weight average molecular weight by the GPC method was 48000.

A dichloromethane solution containing 5 wt% of the obtained purified copolymer was developed on a plate, to obtain an about 30 micrometer thick cast film. The film was immersed in purified water for 24 hours, and it was found to have a water content of 310%, an elastic modulus in tension of 6 MPa and a tensile elongation of 9%. The elastic modulus in tension of the cast film in the dry state was 380 MPa.

The aforesaid purified copolymer was dissolved into dichloromethane, and the method of drying in an O/W emulsion was used to obtain spherical particles. The spherical particles were dried in vacuum and then fractionated using a nylon mesh. The fractionated particles were immersed in a physiologic saline, to obtain a spherical particle dispersion with a volume average particle size of 360 mm and a distribution width of average particle size ± 30 micrometer. The water swelling ratios of ten particles with a diameter of about 360 micrometer immersed in purified water were measured, and a water swelling ratio of 246% was obtained.

The aforesaid spherical particle dispersion was injected from a syringe into a catheter, Mass Transit (overall length about 1400 mm, length of tip portion 180 mm, inner diameter of tip portion about 680 micrometer) produced by Cordis. It could be injected without any resistance. Subsequently, the catheter was dissected in the longitudinal direction, and the inside of the catheter was visually observed. No remaining spherical particle was observed at all.

Next, the blocking of a thin catheter used as a blood vessel model was examined. The spherical particle dispersion was injected from a syringe into a catheter, Spinnaker 1.5F (overall length about 1650 mm, length of tip portion 150 mm, inner diameter of tip portion about 280 micrometer) produced by Boston Scientific, and immediately after start of injection, a strong resistance acted not to allow injection. No particle that passed through the tip portion was observed.

Meanwhile, said spherical particles were added into the phosphate buffered saline (pH 7.3), and after lapse of 28 days at 37°C, the remaining weight in comparison with the weight before treatment was obtained and found to be 35%.

[Example 5]

Under a nitrogen stream, 23.1 g of D,L-lactide (produced by Purac Biochem), 11.5 g of glycolide (produced by Purac Biochem) and 23.1 g of a dehydrated tetra-branched poly(ethylene glycol) derivative with an average molecular weight of 20000, "Sunbright PTE-20000" (produced by NOF Corp.) were mixed in a flask, and the mixture was dissolved and mixed at 140°C. Then, 8.1 mg of tin dioctanoate (produced by Wako Pure Chemical Industries, Ltd.) was added at 180°C to perform a reaction for obtaining poly(D,L-lactide/glycolide)x4-poly(ethylene glycol) copolymer. The copolymer was dissolved into chloroform, and the solution was added dropwise into a very excessive amount of methanol, to obtain a precipitate. The weight average molecular weight by the GPC method was 62000.

A dichloromethane solution containing 5 wt% of the obtained purified copolymer was developed on a plate, to obtain an about 30 micrometer thick cast film. The film was immersed in purified water for 24 hours, and it was found to have a water content of 330%, an elastic modulus in tension of 4 MPa and a tensile elongation of 45%. Meanwhile, the elastic modulus in tension of the cast film in the dry state was 25 MPa.

The aforesaid purified copolymer was dissolved into dichloromethane, and the method of drying in an O/W emulsion was used to obtain spherical particles. The spherical particles were dried in vacuum and then fractionated using a nylon mesh. The fractionated particles were immersed into a physiologic saline, to obtain a spherical particle dispersion with a volume average particle size of 360 micrometer and a distribution width of average particle size ± 40 micrometer. The water swelling ratios of ten particles with a diameter of about 350 micrometer immersed in purified water were measured, and a water swelling ratio of 251% was obtained.

The aforesaid spherical particle dispersion was injected from a syringe into a catheter, Mass Transit (overall length about 1400 mm, length of tip portion 180 mm, inner diameter of tip portion about 680 micrometer) produced by Cordis. It could be injected without any resistance. Subsequently, the catheter was dissected in the longitudinal direction, and the inside of the catheter was visually observed. No remaining spherical particle was observed at all.

Next, the blocking of a fine catheter used as a blood vessel model was examined. The spherical particle dispersion was injected from a syringe into a catheter, Spinnaker 1. 5F (overall length about 1650 mm, length of tip portion 150 mm, inner diameter of tip portion about 280 micrometer) produced by Boston Scientific, and immediately after start of injection, a strong resistance acted not to allow injection. No particle that passed through the tip portion was observed.

Meanwhile, said spherical particles were added into the phosphate buffered saline (pH 7.3), and after lapse of 28 days at 37°C, the remaining weight in comparison with the weight before treatment was obtained and found to be 40%.

[Example 6]

Under a nitrogen stream, 34.6 g of L-lactide (produced by Purac Biochem) and 23.1 g of a dehydrated octa-branched poly(ethylene glycol) derivative with an average molecular weight of 20000, "Sunbright HGEO-20000" (produced by NOF Corp.) were mixed in a flask, and the mixture was dissolved and mixed at 140°C. Then, 8.1 mg of tin dioctanoate (produced by Wako Pure Chemical Industries, Ltd.) was added at 180°C, to perform a reaction, for obtaining a copolymer L with a structure of poly(L-lactide)x8-poly(ethylene glycol). The copolymer was dissolved into chloroform, and the solution was added dropwise into a very excessive amount of methanol, to obtain a white precipitate. The weight average molecular weight by the GPC method was 65000.

Under a nitrogen stream, 34.6 g of D-lactide (produced by Purac Biochem) and 23.1 g of a dehydrated octa-branched poly(ethylene glycol) derivative with an average molecular weight of 20000, "Sunbright HGEO-20000" (produced by NOF Corp.) were mixed in a flask, and the mixture was dissolved and mixed at 140°C. Then, 8.1 mg of tin dioctanoate (produced by Wako Pure Chemical Industries, Ltd.) was added at 180°C, to perform a reaction, for obtaining a copolymer D with a structure of poly(D-lactide)x8-poly(ethylene glycol). The copolymer was dissolved into chloroform, and the solution was added dropwise into a very excessive amount of methanol, to obtain a white precipitate. The weight average molecular weight by the GPC method was 65000.

A dichloromethane solution containing 5 wt% of the purified copolymer L and a dichloromethane solution containing 5 wt% of the purified copolymer D were mixed at a ratio by weight of 1:1 to obtain a blended solution, and it was developed on a plate, to obtain an about 30 micrometer thick cast film. The thermal properties of the cast film were measured using a differential scanning calorimeter, "DSC6200" produced by Seiko Instruments Inc. The melting peak attributable to the poly(L-lactide) of the copolymer L was 148°C, and the melting peak attributable to the poly(D-lactide) of the copolymer D was 148°C. On the contrary, the melting peak attributable to the blend consisting of the copolymer L and the copolymer D was 198°C, about 50°C higher, and no peak was observed at 148°C. From the results, it was judged that the blend formed a stereo complex. The film was immersed in purified water for 24 hours, and it was found to have a water content of 123%, an elastic modulus in tension of 21 MPa and a tensile elongation of 12%. The elastic modulus in tension of the cast film in the dry state was 100 MPa.

The aforesaid purified copolymer was dissolved into dichloromethane, and the method of drying in an O/W emulsion was used to obtain spherical particles. The spherical particles were dried in vacuum and then fractionated using a nylon mesh. The fractionated particles were immersed in a physiologic saline, to obtain a spherical particle dispersion with a volume average particle size of 340 micrometer and a distribution width of average particle size ± 40 micrometer. The water swelling ratios of ten particles with a diameter of about 340 mm immersed in purified water were measured, and a water swelling ratio of 152% was obtained.

The aforesaid spherical particle dispersion was injected from a syringe into a catheter, Mass Transit (overall length about 1400 mm, length of tip portion 180 mm, inner diameter of tip portion about 680 micrometer) produced by Cordis. It could be injected without any resistance. Subsequently, the catheter was dissected in the longitudinal direction, and the inside of the catheter was visually observed. No remaining spherical particle could be observed at all.

Next, the blocking of a fine catheter used as a blood vessel model was examined. The spherical particle dispersion was injected from a syringe into a catheter, Spinnaker 1.5F (overall length about 1650 mm, length of tip portion 150 mm, inner diameter of tip portion about 280 micormeter) produced by Boston Scientific, and immediately after start of injection, a strong resistance acted not to allow injection. No particle that passed through the tip portion was observed.

Meanwhile, said spherical particles were added into the phosphate buffered saline (pH 7.3), and after lapse of 28 days at 37°C, the remaining weight in comparison with the weight before treatment was obtained and found to be 43%.

[Example 7]

Under a nitrogen stream, 30.3 g of L-lactide (produced by Purac Biochem), 10.0 g of glycolide (produced by Purac Biochem) and 17.3 g of dehydrated poly(ethylene glycol) with an average molecular weight of 20000 (produced by Sanyo Chemical Industries, Ltd.) were mixed in a flask, and the mixture was dissolved and mixed at 140°C. Then, 8.1 mg of tin dioctanoate (produced by Wako Pure Chemical Industries, Ltd.) was added at 180°C, to perform a reaction, for obtaining poly(lactide/glycolide)-poly(ethylene glycol)-poly(lactide/glycolide) copolymer. The copolymer was dissolved into chloroform, and the solution was added dropwise into a very excessive amount of methanol, to obtain a white precipitate. The weight average molecular weight by the GPC method was 72000.

The obtained purified copolymer and the purified copolymer obtained in Example 4 were mixed at a ratio by weight of 9:1, and a dichloromethane solution containing 5 wt% of the mixture was developed on a plate, to obtain an about 30 mm thick cast film. The film was immersed in purified water for 24 hours, and it was found to have a water content of 223%, an elastic modulus in tension of 40 MPa and a tensile elongation of 100%. Meanwhile, the elastic modulus in tension of the cast film in the dry state was 90 MPa.

The aforesaid copolymer mixture was dissolved into dichloromethane, and the method of drying in an O/W emulsion was used to obtain spherical particles. The spherical particles were dried in vacuum, and then fractionated using a nylon mesh. The fractionated particles were immersed into a physiologic saline to obtain a spherical particle dispersion with a volume average particle size of 350 micrometer and a distribution width of average particle size ± 40 mm. The water swelling ratios of ten particles with a diameter of about 350 micrometer immersed in pure water were measured, and a water swelling ratio of 210% was obtained.

The aforesaid spherical particle dispersion was injected from a syringe into a catheter, Mass Transit (overall length about 1400 mm, length of tip portion 180 mm, inner diameter of tip portion about 680 mm) produced by Cordis. It could be injected without any resistance. Subsequently, the catheter was dissected in the longitudinal direction, and the inside of the catheter was visually observed. No remaining spherical particle could be observed at all.

Next, the blocking of a thin catheter used as a blood vessel model was examined. The spherical particle dispersion was injected from a syringe into a catheter, Spinnaker 1.5F (overall length about 1650 mm, length of tip portion 150 mm, inner diameter of tip portion about 280 micrometer) produced by Boston Scientific, and immediately after start of injection, a strong resistance acted not to allow injection. No particle that passed through the tip portion was observed.

Meanwhile, said spherical particles were added into the phosphate buffered solution (pH 7.3), and after lapse of 28 days at 37°C, the remaining weight in comparison with the weight before treatment was obtained and found to be 55%.

INDUSTRIAL APPLICABILITY

As described above, the embolization material of this invention can reliably block a blood vessel at an intended site without causing aggregation or clogging in a catheter or in the blood vessel at other than the intended site. Furthermore, irrespective of the blocking site and the blocking environment, the blocked site concerned can be liberated from the embolized state with the lapse of time, and the embolization material is degraded finally in vivo. The degraded components can be metabolized or excreted outside the body. Therefore, it can be suitably used in the field of medical therapy, as a hemorrhage preventing means for a surgical operation, as arterial embolization to stop the supply of nutrition to a tumor incapable of being excised, and as chemo -embolization therapy in which the anticancer drug concentration in a tumor can be kept high.

**Claims**

1. An embolization material which contains a synthetic polymer, has a water swelling ratio of 30% or more, is degradable in a phosphate buffered saline at 37°C, and is formed as virtually spherical particles with an average particle size of 50 $\mu$m or more, wherein the synthetic polymer is a water insoluble poly(ethylene glycol) copolymer having a structure in which a poly(ethylene glycol) derivative which is selected from poly (ethylene glycol), methoxy poly (ethylene glycol) and multi-branched poly (ethylene glycol) and a biodegradable polymer are chemically bonded to each other, and is capable of forming a film which, when saturated with water, has an elastic modulus in tension of 1500 MPa or less, and wherein the water insoluble poly(ethylene glycol) copolymer has a weight average molecular weight of 3000 to 100000, and the structure of the poly(ethylene glycol) derivative existing in the poly(ethylene glycol) copolymer has a weight average molecular weight of 2000 to 50000.

2. An embolization material according to claim 1, wherein the film, when saturated with water, has an elastic modulus in tension of 4 to 400 MPa.

3. An embolization material according to claim 1 or 2, wherein the elastic modulus in tension of the film, when saturated with water, is 60% or less of the elastic modulus in tension of the film in the dry state.

4. An embolization material according to any one of claims 1 to 3, wherein the film, when saturated with water, has a tensile elongation of 100% or more.

5. An embolization material according to any one of claims 1 to 4, which has a water swelling ratio of 100% or more.

6. An embolization material according to any one of claims 1 to 5, wherein the weight of the water insoluble polymer remaining after immersion thereof in a phosphate buffered saline at 37°C for 28 days is 80% or less of the weight thereof prior to immersion.

**Patentansprüche**

1. Embolisationsmaterial, das ein synthetisches Polymer enthält, ein Wasserquellverhältnis von 30 % oder mehr aufweist, in phosphatgepufferter Salzlösung bei 37 °C abbaubar ist und in Form von praktisch kugelförmigen Teilchen mit einer mittleren Teilchengröße von 50 μm oder mehr vorliegt, worin das synthetische Polymer ein wasserlösliches Poly(ethylenglykol)-Copolymer ist, das eine Struktur aufweist, in der ein Poly(ethylenglykol)-Derivat, das aus Poly(ethylenglykol), Methoxypoly(ethylenglykol) und mehrfach verzweigtem Poly(ethylenglykol) ausgewählt ist, und ein biologisch abbaubares Polymer chemisch miteinander verbunden sind, und das in der Lage ist, einen Film zu bilden, der, wenn er mit Wasser gesättigt ist, einen Elastizitätsmodul von 1.500 MPa oder weniger aufweist, und worin das wasserunlösliche Poly(ethylenglykol)-Copolymer ein gewichtsmittleres Molekulargewicht von 3.000 bis 100.000 aufweist und die Struktur des Poly(ethylenglykol)-Derivats, das in dem Poly(ethylenglykol)-Copolymer vorliegt, ein gewichtsmittleres Molekulargewicht von 2.000 bis 50.000 aufweist.

2. Embolisationsmaterial nach Anspruch 1, worin der Film, wenn er mit Wasser gesättigt ist, einen Elastizitätsmodul von 4 bis 400 MPa aufweist.

3. Embolisationsmaterial nach Anspruch 1 oder 2, worin der Zug-Elastizitätsmodul des Films, wenn er mit Wasser gesättigt ist, 60 % oder weniger des Zug-Elastizitätsmodul des Films in trockenem Zustand entspricht.

4. Embolisationsmaterial nach einem der Ansprüche 1 bis 3, worin der Film, wenn er mit Wasser gesättigt ist, eine Zugdehnung von 100 % oder mehr aufweist.

5. Embolisationsmaterial nach einem der Ansprüche 1 bis 4, das ein Wasserquellverhältnis von 100 % oder mehr aufweist.

6. Embolisationsmaterial nach einem der Ansprüche 1 bis 5, worin das Gewicht des wasserunlöslichen Polymers, das nach Einlegen in eine phosphatgepufferte Salzlösung bei 37 °C für 28 Tage beibehalten wird, 80 % oder weniger seines Gewichts vor dem Einlegen ausmacht.

## Revendications

1. Matériau d'embolisation qui contient un polymère synthétique, présente un rapport de gonflement à l'eau de 30 % ou plus, est dégradable dans une solution saline tamponnée au phosphate à 37 °C, et se présente sous la forme de particules quasiment sphériques ayant une granulométrie moyenne de 50 μm ou plus, dans lequel le polymère synthétique est un copolymère de poly(éthylène glycol) insoluble dans l'eau ayant une structure dans laquelle un dérivé de poly(éthylène glycol) qui est choisi parmi le poly (éthylène glycol), le méthoxy poly (éthylène glycol) et le poly (éthylène glycol) à ramifications multiples et un polymère biodégradable sont liés chimiquement l'un à l'autre, et est capable de former un film qui, lorsqu'il est saturé avec de l'eau, présente un module élastique en tension de 1 500 MPa ou moins, et dans lequel le copolymère de poly(éthylène glycol) insoluble dans l'eau a un poids moléculaire moyen en poids de 3 000 à 100 000, et la structure du dérivé de poly(éthylène glycol) existant dans le copolymère de poly(éthylène glycol) a un poids moléculaire moyen en poids de 2 000 à 50 000.

2. Matériau d'embolisation selon la revendication 1, dans lequel le film, lorsqu'il est saturé avec de l'eau, présente un module élastique en tension de 4 à 400 MPa.

3. Matériau d'embolisation selon la revendication 1 ou 2, dans lequel le module élastique en tension du film, lorsqu'il est saturé avec de l'eau, est de 60 % ou moins du module élastique en tension du film à l'état sec.

4. Matériau d'embolisation selon l'une quelconque des revendications 1 à 3, dans lequel le film, lorsqu'il est saturé avec de l'eau, présente un allongement en traction de 100 % ou plus.

5. Matériau d'embolisation selon l'une quelconque des revendications 1 à 4, qui présente un rapport de gonflement à l'eau de 100 % ou plus.

6. Matériau d'embolisation selon l'une quelconque des revendications 1 à 5, dans lequel le poids du polymère insoluble dans l'eau restant après immersion de celui-ci dans une solution saline tamponnée au phosphate à 37 °C pendant 28 jours est de 80 % ou moins du poids de celui-ci avant immersion.

**Patent documents cited in the description**

- JP 2000502321 T
- JP 6056676 A
- US 5635215 A
- JP 6508139 T
- JP 2000505045 T
- JP 2000506514 T
- US 2002111392 A
- WO 0172280 A
- WO 0180713 A
- JP 60020934 A
- JP 60222046 A
- WO 3103368 A
- JP 6329542 A
- JP 10003203 A
- JP 5000969 A
- JP 5017245 B

**Non-patent literature cited in the description**

- **M. KODA et al.** *Cancer,* 2001, vol. 92 (6), 1516-1524
- Cyanoacrylate described in the n-BCA Trial Investigators. *American Journal of Neuroradiology,* 2002, vol. 23 (5), 748-755
- **M. ANTHONY et al.** *American Journal of Neuroradiology,* 2000, vol. 21 (8), 1523-1531
- **C.P. DARDEYN.** *American Journal of Neuroradiology,* 1995, vol. 16, 1335-1343
- **T. KUMADA et al.** *Nihon-Rinsho,* 2001, vol. 59 (6), 539-544
- **C. WITTE et al.** *European Journal of Pharmaceutics and Biopharmaceutics,* 2001, vol. 51, 171-181